# EUROPEAN PATENT SPECIFICATION

(11) **EP 0 912 239 B1**
(45) Date of publication and mention of the grant of the patent: **26.09.2001**
(21) Application number: 97926146.8
(22) Date of filing: 17.06.1997
(51) Int. Cl.: B01J 2/22, A61K 41/00

(54) **METHOD FOR PROVIDING DENSE PARTICLE COMPOSITIONS FOR USE IN TRANSDERMAL PARTICLE DELIVERY**
VERFAHREN ZUR LIEFERUNG VON ZUSAMMENSETZUNGEN AUS DICHTEN TEILCHEN ZUR ANWENDUNG IN TRANSDERMALE TEILCHEN ABGABE
PROCEDE D'OBTENTION DE COMPOSITIONS EN PARTICULES DENSES POUR ADMINISTRATION PAR VOIE TRANSDERMIQUE

(30) Priority: 17.06.1996 GB 9612629
(43) Date of publication of application: 06.05.1999
(73) Proprietor: PowderJect Research Limited, Oxford OX4 4GA (GB)
(72) Inventor: SARPHIE, David, Francis, Madison WI 53719 (US); BURKOTH, Terry, Lee, Palo Alto, CA 94303 (US)
(74) Representative: Woods, Geoffrey Corlett
(86) International application number: GB9701636
(87) International publication number: WO9748485

(56) References cited:
- EP-A- 0 446 597
- US-A- 5 147 291
- BIOELECTROCHEMISTRY AND BIOENERGITICS, vol. 38, no. 1, 1995, pages 209-222, XP000618249 HOFMANN, GÜNTER ET ALL: "electro-incorporation of microcarries as a method for the transdermal delivery of large molecules"

## Description

### Technical Field

The invention relates to a method for densifying processed pharmaceutical compositions.

### Background of the Invention

The ability to deliver drugs through skin surfaces (transdermal delivery) provides many advantages over oral or parenteral delivery techniques. In particular, transdermal delivery provides a safe, convenient and noninvasive alternative to traditional drug administration systems, conveniently avoiding the major problems associated with oral delivery, e.g., variable rates of absorption and metabolism, gastrointestinal irritation and/or bitter or unpleasant drug tastes. Transdermal delivery also avoids problems associated with parenteral delivery, e.g., needle pain, the risk of introducing infection to treated individuals, the risk of contamination or infection of health care workers caused by accidental needle-sticks and the disposal of used needles. In addition, such delivery affords a high degree of control over blood concentrations of administered drugs.

However, despite its clear advantages, transdermal drug delivery presents a number of its own inherent logistical problems. The passive delivery of drugs through intact skin necessarily entails the transport of molecules through a number of structurally different tissues, including the stratum corneum, the viable epidermis, the papillary dermis, and the capillary walls in order for the drug to gain entry into the blood or lymph system. Transdermal delivery systems must therefore be able to overcome the various resistances presented by each type of tissue. In light of the above, a number of alternatives to passive transdermal delivery have been developed. These alternatives include the use of skin penetration enhancing agents, or "permeation enhancers," to increase skin permeability, as well as non-chemical modes such as the use of iontophoresis, electroporation or ultrasound. However, such techniques often give rise to unwanted side effects, such as skin irritation or sensitization. Thus, the number of drugs that can be safely and effectively administered using traditional transdermal delivery methods has remained limited.

More recently, a novel transdermal drug delivery system that entails the use of a needleless syringe to fire solid drug-containing particles in controlled doses into and through intact skin has been described. In particular, commonly assigned U.S. Patent Application Serial Number 08/474,367, entitled "Transdermal Drug Delivery" and filed June 7, 1995 by Bellhouse et al. (now US-A-5 630 796) describes a needleless syringe that delivers pharmaceutical particles entrained in a supersonic gas flow. The needleless syringe is used for transdermal delivery of powdered drug compounds and compositions, for delivery of genetic material into living cells (e.g., gene therapy) and for the delivery of biopharmaceuticals to skin, muscle, blood or lymph. The needleless syringe can also be used in conjunction with surgery to deliver drugs and biologics to organ surfaces, solid tumors and/or to surgical cavities (e.g., tumor beds or cavities after tumor resection). In theory, practically any pharmaceutical agent that can be prepared in a substantially solid, particulate form can be safely and easily delivered using such devices.

One particular needleless syringe generally comprises an elongate tubular nozzle having a rupturable membrane initially closing the passage through the nozzle and arranged substantially adjacent to the upstream end of the nozzle. Particles of a therapeutic agent to be delivered are disposed adjacent to the rupturable membrane and are delivered using an energizing means which applies a gaseous pressure to the upstream side of the membrane sufficient to burst the membrane and produce a supersonic gas flow (containing the pharmaceutical particles) through the nozzle for delivery from the downstream end thereof. The particles can thus be delivered from the needleless syringe at delivery velocities of between Mach 1 and Mach 8 which are readily obtainable upon the bursting of the rupturable membrane.

Another needleless syringe configuration generally includes the same elements as described above, except that instead of having the pharmaceutical particles entrained within a supersonic gas flow, the downstream end of the nozzle is provided with a bistable diaphragm which is moveable between a resting "inverted" position (in which the diaphragm presents a concavity on the downstream face to contain the pharmaceutical particles) and an active "everted" position (in which the diaphragm is outwardly convex on the downstream face as a result of a supersonic shockwave having been applied to the upstream face of the diaphragm). In this manner, the pharmaceutical particles contained within the concavity of the diaphragm are expelled at a supersonic initial velocity from the device for transdermal delivery thereof to a targeted skin or mucosal surface.

Transdermal delivery using the above-described needleless syringe configurations is carried out with particles having an approximate size that generally ranges between 0.1 and 250 *µ*m. For drug delivery, an optimal particle size is usually at least about 10 to 15 *µ*m (the size of a typical cell). For gene delivery, an optimal particle size is generally substantially smaller than 10 *µ*m. Particles larger than about 250 *µ*m can also be delivered from the device, with the upper limitation being the point at which the size of the particles would cause untoward damage to the skin cells. The actual distance which the delivered particles will penetrate depends upon particle size (e.g., the nominal particle diameter assuming a roughly spherical particle geometry), particle density, the initial velocity at which the particle impacts the skin surface, and the density and kinematic viscosity of the skin. In this regard, optimal particle densities for use in needleless injection generally range between about 0.1 and 25 g/cm³, preferably between about 0.8 and 1.5 g/cm³, and injection velocities generally range between about 200 and 3,000 m/sec.

A particularly unique feature of the needleless syringe is the ability to closely control the depth of penetration of delivered particles, thereby allowing for targeted administration of pharmaceuticals to various sites. For example, particle characteristics and/or device operating parameters can be selected to provide penetration depths of less than about 1 mm for intra-dermal delivery, or approximately 1-2mm for subcutaneous delivery. One approach entails the selection of particle size, particle density and initial velocity to provide a momentum density (e.g., particle momentum divided by particle frontal area) of between about 2 and 10 kg/sec/m, and more preferably between about 4 and 7 kg/sec/m. Such control over momentum density allows for precisely controlled, tissue-selective delivery of the pharmaceutical particles.

Accordingly, there is a need to provide a reliable method for preparing sufficiently dense particles (having a density of about 0.8 to 1.5 g/cm³) which have an average size of about 0.1 to 150 µm from a wide variety of pharmaceutical compositions. The densified pharmaceutical particles can thus be transdermally delivered to a subject using a needleless syringe system.

### Summary of the Invention

The present invention provides a method for forming densified particles from a particulate pharmaceutical preparation, comprising compacting the preparation to provide a compacted pharmaceutical preparation and size-reducing the compacted preparation into densified particles of suitable size and density for transdermal delivery thereof by needleless injection.

One suitable such method comprises compacting the particulate pharmaceutical preparation to provide a compacted pharmaceutical preparation, size-reducing the compacted preparation into densified particles, and then selecting densified particles of suitable size and density for transdermal delivery thereof by needleless injection. The compacting is generally carried out without heating or shear. The size reduction of the compacted material is typically carried out by milling and/or sieving.

The invention also provides a densified particulate pharmaceutical composition formed from a lyophilized or spray-dried pharmaceutical preparation, said densified composition having an average particle size in the range of 0.1 to 250 µm and a particle density in the range of 0.1 to 25 g/cm³. A needleless syringe loaded with particles of the invention is additionally provided.

In one embodiment of the invention, therefore, a method is provided for converting non-dense pharmaceutical powders or particulate formulations (e.g., having particle densities below that required for transdermal delivery at supersonic velocities) into densified particles that are optimally suited for transdermal delivery using needleless syringe. Such particles have an optimal particle density ranging from 0.1 to about 25 g/cm³, preferably ranging from about 0.5 or 0.8 to about 3.0 g/cm³, and most preferably ranging from about 0.8 to about 1.5 g/cm³. The densified particles are processed to obtain optimal particle sizes ranging from about 0.1 to about 250µm, preferably ranging from about 0.1 to about 150 µm, and most preferably ranging from about 20 to about 60 µm. The method entails the compaction of a pharmaceutical composition using high pressure and, optionally vacuum, to densify the composition. The resulting compacted material is then size-reduced using conventional methods to provide densified particles of optimized size.

In a related embodiment of the invention, a method is provided for optimizing the density and particle size of a particulate pharmaceutical composition that has particle size and density characteristics that fall within the above ranges using the above-described compaction and size-reduction techniques. In this manner, the penetration depths that are obtained when the optimized particles are delivered at supersonic velocities using a needleless syringe can be adjusted to provide targeted intra-dermal delivery (e.g., the particle size and density are optimized for penetration of less than about 1 mm) or sub-cutaneous delivery (e.g., the particle size and density are optimized for penetration of approximately 1-2 mm).

In a further related embodiment, the invention pertains to a method for optimizing the particle size and density of a lyophilized or spray-dried biopharmaceutical composition. The method entails the compaction of a lyophilized or spray-dried pharmaceutical powder to obtain a densified material. The densified material can then be reground to produce compositions in which the individual particles approach the theoretical maximum density and are thus optimal for delivery by impact with and penetration into skin at supersonic velocities when delivered from a needleless syringe. In a particular embodiment, lyophilized recombinant human growth hormone (rhGH) powder is densified to obtain particles in the range of about 20 to 50 *µ* m and having a density of about 0.8 to 1.5 g/cc³. The densified rhGH particles are ideally suited for delivery from a needleless syringe device.

These and other embodiments of the invention will readily occur to those of ordinary skill in the art in view of the disclosure herein.

### Brief Description of the Drawings

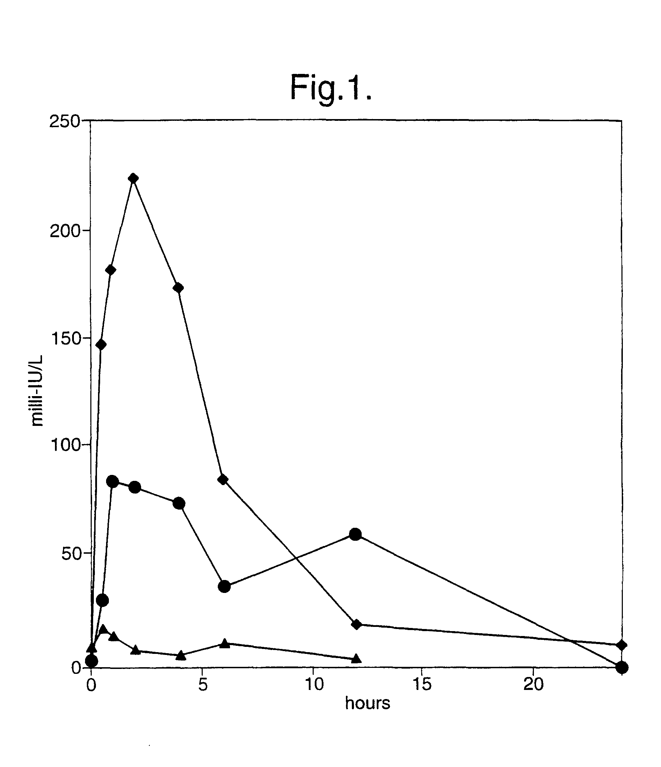

Figure 1 depicts a comparison of the mean *in vivo* serum levels of recombinant human growth hormone (rhGH) in animals that were administered lyophilized rhGH powder by needleless injection (▲), densified rhGH particles (prepared by the method of the invention) by needleless injection (■), or lyophilized rhGH powder by sub-cutaneous injection (•).

### Detailed Description of the Preferred Embodiments

Before describing the present invention in detail, it is to be understood that this invention is not limited to particular pharmaceutical formulations or process parameters as such may, of course, vary. It is also to be understood that the terminology used herein is for the purpose of describing particular embodiments of the invention only, and is not intended to be limiting.

All publications, patents and patent applications cited herein, whether *supra* or *infra,* are hereby incorporated by reference in their entirety.

It must be noted that, as used in this specification and the appended claims, the singular forms "a", "an" and "the" include plural referents unless the content clearly dictates otherwise. Thus, for example, reference to "a pharmaceutical agent" includes a mixture of two or more pharmaceutical agents, reference to "an excipient" includes mixtures of two or more excipients, and the like.

### A. Definitions

Unless defined otherwise, all technical and scientific terms used herein have the same meaning as commonly understood by one of ordinary skill in the art to which the invention pertains. Although a number of methods and materials similar or equivalent to those described herein can be used in the practice of the present invention, the preferred materials and methods are described herein.

In describing the present invention, the following terms will be employed, and are intended to be defined as indicated below.

By "transdermal" delivery, applicants intend to include both transdermal (or "percutaneous") and transmucosal administration, i.e., delivery by passage of a drug or pharmaceutical agent through the skin or mucosal tissue. *See, e.g*., *Transdermal Drug Delivery: Developmental Issues and Research Initiatives,* Hadgraft and Guy (eds.), Marcel Dekker, Inc., (1989); *Controlled Drug Delivery: Fundamentals and Applications,* Robinson and Lee (eds.), Marcel Dekker Inc., (1987); and *Transdermal Delivery of Drugs,* Vols. 1-3, Kydonieus and Berner (eds.), CRC Press, (1987). Aspects of the invention which are described herein in the context of "transdermal" delivery, unless otherwise specified, are meant to apply to both transdermal and transmucosal delivery. That is, the compositions, systems, and methods of the invention, unless explicitly stated otherwise, should be presumed to be equally applicable with transdermal and transmucosal modes of delivery.

As used herein, the term "drug" or "pharmaceutical agent" intends any compound or composition of matter which, when administered to an organism (human or animal) induces a desired pharmacologic and/or physiologic effect by local and/or systemic action. The term therefore encompasses those compounds or chemicals traditionally regarded as drugs, as well as biopharmaceuticals including molecules such as peptides, hormones, nucleic acids, gene constructs and the like. More particularly, the term "drug" or "pharmaceutical agent" includes compounds or compositions for use in all of the major therapeutic areas including, but not limited to, anti-infectives such as antibiotics and antiviral agents; analgesics and analgesic combinations; local and general anesthetics; anorexics; antiarthritics; antiasthmatic agents; anticonvulsants; antidepressants; antihistamines; anti-inflammatory agents; antinauseants; antineoplastics; antipruritics; antipsychotics; antipyretics; antispasmodics; cardiovascular preparations (including calcium channel blockers, beta-blockers, beta-agonists and antiarrythmics); antihypertensives; diuretics; vasodilators; central nervous system stimulants; cough and cold preparations; decongestants; diagnostics; hormones; bone growth stimulants and bone resorption inhibitors; immunosuppressives; muscle relaxants; psychostimulants; sedatives; tranquilizers; proteins peptides and fragments thereof (whether naturally occurring, chemically synthesized or recombinantly produced); and nucleic acid molecules (polymeric forms of two or more nucleotides, either ribonucleotides (RNA) or deoxyribonucleotides (DNA) including both double- and single-stranded molecules, gene constructs, expression vectors, antisense molecules and the like).

The above drugs or pharmaceutical agents, alone or in combination with other drugs or agents, are typically prepared as pharmaceutical compositions which can contain one or more added materials such as carriers, vehicles, and/or excipients. "Carriers," "vehicles" and "excipients" generally refer to substantially inert materials which are nontoxic and do not interact with other components of the composition in a deleterious manner. These materials can be used to increase the amount of solids in particulate pharmaceutical compositions, such as those prepared using spray-drying or lyophilization techniques. Examples of suitable carriers include water, silicone, gelatin, waxes, and like materials. Examples of normally employed "excipients," include pharmaceutical grades of dextrose, sucrose, lactose, trehalose, mannitol, sorbitol, inositol, dextran, starch, cellulose, sodium or calcium phosphates, calcium sulfate, citric acid, tartaric acid, glycine, high molecular weight polyethylene glycols (PEG), and combinations thereof.

"Gene delivery" refers to methods or systems for reliably inserting foreign DNA into host cells. Such methods can result in expression of non-integrated transferred DNA, extrachromosomal replication and expression of transferred replicons (e.g., episomes), or integration of transferred genetic material into the genomic DNA of host cells.

By "vector" is meant any genetic element, such as a plasmid, phage, transposon, cosmid, chromosome, virus, virion, etc., which is capable of replication when associated with the proper control elements and which can transfer gene sequences between cells.

### B. General Methods

In one embodiment, the invention entails a procedure for forming dense particles from low density particulate pharmaceutical preparations. In particular, manufacturing processes for preparing pharmaceutical particles from delicate molecules such as proteins or peptides generally result in low density particles having either a hollow spherical or open lattice monolithic structure. Such particles are poorly suited for use in needleless syringe delivery systems, wherein the particles must have sufficient physical strength to withstand sudden acceleration to several times the speed of sound and the impact with, and passage through, the skin and tissue.

One common method of preparing pharmaceuticals, particularly heat-sensitive biopharmaceutical particles, is lyophilization (freeze-drying). Lyophilization relates to a technique for removing moisture from a material and involves rapid freezing at a very low temperature, followed by rapid dehydration by sublimation in a high vacuum. This technique typically yields low-density porous particles having an open matrix structure. Such particles are chemically stable, but are rapidly reconstituted (disintegrated and/or brought into solution) when introduced into an aqueous environment. Exemplary biopharmaceuticals available as lyophilized particles include recombinant human growth factor (e.g., Genotropin® , Pharmacia, Piscataway, NJ); somatrem (e.g., Protropin® , Genentech, S. San Francisco, CA); somatropin (e.g., Humatrope® , Eli Lilly, Indianapolis, IN); recombinant interferon α-2a (e.g., Roferon® -A, Hoffman-La Roche, Nutley, NJ); recombinant interferon α-2b (e.g., Intron A® , Schering-Plough, Madison, NJ); and recombinant alteplase (e.g., Activase® , Genentech, S. San Francisco, CA).

Another method of preparing pharmaceutical particles that is often used with delicate or heat-sensitive biomolecules is spray-drying. Spray-drying relates to the atomization of a solution of one or more solids using a nozzle, spinning disk or other device, followed by evaporation of the solvent from the droplets. More particularly, spray-drying involves combining a highly dispersed liquid pharmaceutical preparation (e.g., a solution, slurry, emulsion or the like) with a suitable volume of hot air to produce evaporation and drying of the liquid droplets. Spray-dried pharmaceuticals are generally characterized as homogenous spherical particles that are frequently hollow. Such particles have low density and exhibit a rapid rate of solution. Exemplary heat-sensitive pharmaceuticals that are prepared using spray-drying techniques include the amino acids; antibiotics such as aureomycin, bacitracin, penicillin and streptomycin; ascorbic acid; cascara extracts; pepsin and similar enzymes; protein hydrolysates; and thiamine.

When spray-dried and lyophilized pharmaceutical particles are ground or milled, they yield very small, light and non-dense particles that are poorly suited for delivery through skin or mucosal tissues. In particular, such particles, when delivered from a needleless syringe, are often too light to have the momentum necessary to penetrate intact skin (e.g., cross through the stratum corneum) and would thus fail to enter into systemic circulation. In this regard, the stratum corneum is a thin layer of dense packed, highly keratinized cells, generally about 10-15 *µ*m thick and which covers most of the human body. The stratum corneum thus provides the primary skin barrier which a transdermally-delivered particle must cross.

Accordingly, the present method entails densifying such preparations to provide particles that are much better suited for delivery from a needleless syringe (e.g., substantially solid particles having a size of about 50 *µ*m and a density of at least about 0.8 to 1.5 g/cc³). In particular, the open lattice or hollow shell particles provided by spray-drying or lyophilization are condensed without heating or shear to provide dense materials that can be milled or otherwise size-reduced to yield pharmaceutical particles having both size and density characteristics suitable for delivery by needleless injection.

Condensing of the spray-dried or lyophilized powders is conducted by compaction in a suitable press (e.g., a hydraulic press, tableting press or rotary press), wherein the powders are compressed at about 7.03 x 10⁴ to 1.68 x 10⁶g/cm² (1,000 to 24,000 pounds/square inch, 0.5 to 12 tons/square inch) for a suitable time. Compaction can be carried out under vacuum if desired. The resulting compacted material is then coarsely reground until visually broken up. The particle size is then reduced to about a 20 to 50 *µ*m average size to yield a density of around 0.8 to 1.5 g/cc³. Particle size reduction can be conducted using methods well known in the art including, but not limited to, roller milling, ball milling, hammer or impact milling, attrition milling, sieving, sonicating, or combinations thereof. The compression parameters and particle sizing will, of course, vary depending upon the starting material used, the desired target particle size and density, and like considerations. The starting material can be any pharmaceutical preparation having a particle size and density which one is desirous of changing to obtain more optimal size and density characteristics for use in needleless syringes.

Actual particle density, or "absolute density," can be readily ascertained using known quantification techniques such as helium pycnometry and the like. Alternatively, envelope density measurements can be used to assess suitable densification of the particulate pharmaceutical compositions. Envelope density information is useful in characterizing the density of porous objects of irregular size and shape. Envelope density, or "bulk density," is the mass of an object divided by its volume, where the volume includes that of its pores and small cavities. A number of methods of determining envelope density are known in the art, including wax immersion, mercury displacement, water absorption and apparent specific gravity techniques. A number of suitable devices are also available for determining envelope density, for example, the GeoPyc™ Model 1360, available from the Micromeritics Instrument Corp. The difference between the absolute density and envelope density of a sample pharmaceutical composition provides information about the sample's percentage total porosity and specific pore volume. In the practice of the invention, compaction of porous particulate pharmaceutical compositions will generally result in a reduction of porosity, and a concomitant increase in envelope density.

Thus, the method can be used to obtain particles having a size ranging from about 0.1 to about 250 *µ*m, preferably about 0.1 to about 150 *µ*m, and most preferably about 20 to about 60 *µ*m; and a particle density ranging from about 0.1 to about 25 g/cm³, preferably about 0.5 or 0.8 to about 3.0 g/cm³, and most preferably about 0.8 to about 1.5 g/cm³.

The above-described method can also be used to optimize the density and particle size of a particulate pharmaceutical composition that has particle size and density characteristics that fall within the above ranges. In this manner, the penetration depths that are obtained when the optimized particles are delivered at supersonic velocities using a needleless syringe can be adjusted to provide targeted intra-dermal delivery (e.g., the particle size and density are optimized for penetration of less than about 1 mm) or sub-cutaneous delivery (e.g., the particle size and density are optimized for penetration of approximately 1-2 mm).

However, as noted hereinabove, the invention is particularly suited for preparing densified particles having optimized density from heat-sensitive biopharmaceutical preparations of peptides, polypeptides, proteins and other such biological molecules. Exemplary peptide and protein formulations which can be densified using the instant method include, without limitation, insulin; calcitonin; octreotide; endorphin; liprecin; pituitary hormones (e.g., human growth hormone and recombinant human growth hormone (hGH and rhGH), HMG, desmopressin acetate, etc) ; follicle luteoids; growth factors (such as growth factor releasing factor (GFRF), somatostatin, somatotropin and platelet-derived growth factor); asparaginase; chorionic gonadotropin; corticotropin (ACTH); erythropoietin (EPO); epoprostenol (platelet aggregation inhibitor); glucagon; interferons; interleukins; menotropins (urofollitropin follicle-stimulating hormone (FSH) and luteinizing hormone (LH)); oxytocin; streptokinase; tissue plasminogen activator (TPA); urokinase; vasopressin; desmopressin; ACTH analogues; angiotensin II antagonists; antidiuretic hormone agonists; bradykinin antagonists; CD4 molecules; antibody molecules and antibody fragments (e.g., Fab, Fab₂, Fv and sFv molecules); IGF-1; neurotrophic factors; colony stimulating factors; parathyroid hormone and agonists; parathyroid hormone antagonists; prostaglandin antagonists; protein C; protein S; renin inhibitors; thrombolytics; tumor necrosis factor (TNF); vaccines (particularly peptide vaccines including subunit and synthetic peptide preparations); vasopressin antagonists analogues; and α-1 antitrypsin. Additionally, non-dense preparations of vectors or gene constructs for use in subsequent gene delivery are also particularly well suited for densification using the method of the present invention.

### C. Experimental

Below are examples of specific embodiments for carrying out the present invention. The examples are offered for illustrative purposes only, and are not intended to limit the scope of the present invention in any way.

Efforts have been made to ensure accuracy with respect to numbers used *(e.g.,* amounts, temperatures, etc.), but some experimental error and deviation should, of course, be allowed for.

### Example 1

### Densification of Recombinant

### Human Growth Hormone (rhGH)

Lyophilized recombinant human growth hormone powder (Genotropin® , available from Pharmacia, Piscataway, NJ) was obtained and reprocessed using the method of the invention. Particularly, approximately 30 mg of Genotropin was compacted under pressure using a Carver Laboratory Pellet Press (Model 3620, available from Carver, Inc., Wabash, IN). The pressure of compaction was 1.05 x 10⁶ g/cm² (15,000 lbs/in²) which was applied for approximately 45 seconds. A pellet was obtained which was ground using mortar and pestle until visually broken up. The resulting reduced pellet was then sieved using a 53 *µ*m sieve (Endecott, London). Particles having a size greater 53 *µ* m were selected and appropriate dosages thereof were measured into drug cassettes for delivery from a needleless syringe.

### Example 2

### Visual Assessment of rhGH Particle Penetration

Lyophilized recombinant human growth hormone (rhGH) powder, and densified rhGH particles prepared as described in Example 1 were administered to porcine subjects using a needleless syringe. The degree of particle penetration was visually ascertained as follows.

Genotropin® 36 IU lyophilized powder was milled gently, weighed into individual doses of approximately 0.8 mg powder and loaded into a needleless syringe device for delivery. Densified Genotropin® was prepared as described in Example 1 and approximately 0.8 mg of the densified particles were loaded into a needleless syringe device for delivery.

Porcine subjects were prepared by clipping a sufficient area on the hindquarters. The lyophilized powder and densified particles were delivered to the porcine skin under high velocity. Upon a side-by-side comparison, it was observed that a higher proportion of the densified particles penetrated the skin as evidenced by the visual presence of the lyophilized powder remaining largely on the surface of the skin while substantially no densified particles were observed to remain on the surface of the skin.

### Example 3

### Serum Levels of Transdermally-Delivered rhGH

In order to determine the efficiency with which densified rhGH is delivered using a needleless syringe system, the following study was carried out. Three groups of 5 healthy New Zealand White rabbits were prepared by clipping the fur from the flank area to expose a sufficient area for delivery of lyophilized recombinant human growth hormone powder (rhGH) or densified rhGH by needleless syringe.

Approximately 0.8 mg of lyophilized Genotropin® powder was reconstituted into 1.8 mL of a suitable buffer without preservatives (e.g., sterile phosphate buffered saline (PBS)) to provide a Genotropin® solution having a concentration of 20 IU/mL. 1 mL dosages were withdrawn by syringe and gently mixed with 1 mL of buffer to provide an injection solution having a concentration of 10 IU/mL.

Each animal in the first group were given 0.1 mL/kg of the injection solution by subcutaneous injection, and the injection site was observed to ensure that there was no leakage of the injected solution after administration. Venous blood samples were taken from the marginal ear vein of the right ear of each animal at 0, 30 minutes, 1, 2, 4, 6, 12 and 24 hours after administration. Serum levels of rhGH were ascertained using an immunoassay with labeled anti-rhGH antibodies. The mean serum levels of subcutaneously delivered Genotropin® (•) found in the animals of Group 1 at each time point are depicted in Figure 1.

Approximately 2 mg of lyophilized Genotropin® powder was loaded into a needleless syringe. The lyophilized powder was administered to each animal in the second group by needleless injection at high velocity. Venous blood samples were taken from the marginal ear vein of the right ear of each animal at 0, 30 minutes, 1, 2, 4, 6, 12 and 24 hours after administration. Serum levels of the administered rhGH were ascertained using an immunoassay with labeled anti-hGH antibodies. The mean serum levels of transdermally injected lyophilized Genotropin® powder (▲) found in the animals of Group 2 at each time point are depicted in Figure 1.

Approximately 2 mg of densified Genotropin® particles prepared as in Example 1 was loaded into a needleless syringe. The densified particles were administered to each animal in the third group by needleless injection at high velocity. Venous blood samples were taken from the marginal ear vein of the right ear of each animal at 0, 30 minutes, 1, 2, 4, 6, 12 and 24 hours after administration. Serum levels of the administered rhGH were ascertained using an immunoassay with labeled anti-rhGH antibodies. The mean serum levels of transdermally injected densified Genotropin® particles (■) found in the animals of Group 3 at each time point are depicted in Figure 1.

As can be seen, markedly increased blood serum levels of the densified Genotropin® particles administered by needleless syringe were obtained as compared to the lyophilized Genotropin® powder.

### Example 4

### Determination of Optimum Conditions

### for Needleless Syringe Delivery of rhGH

In order to determine optimum conditions for delivery of rhGH using a needleless syringe delivery system, the following study is carried out. One group of 8 healthy New Zealand White rabbits (2 ± 0.25 kg) are prepared by clipping the fur from the flank area to expose a sufficient area for delivery of Lyophilized recombinant human growth hormone powder (rhGH) or densified rhGH by needleless syringe, sub-cutaneous (SC) or intravenous (IV) injection. The animals are weighed at the start of the study and on a weekly basis throughout the study to determine appropriate Genotropin® dosages. The animals remain in one large group to obtain statistically-significant data.

For an initial needleless syringe injection series, Genotropin® 36 IU lyophilized powder is milled gently and filled into a glass vial. The milled lyophilized powder is weighed into individual dosages and loaded into needleless syringe devices at approximately 0.8 mg powder/kg. The injection is conducted in the first week of the study, and multiple venous blood samples (1 mL whole blood) are taken from the marginal ear vein at times 0, 30 minutes, 1, 2, 4, 6, 12, 24 and 48 hours after administration. The animals are individually-housed at all times with food and water available *ad libitum.*

Blood samples are handled and processed as follows: each venous blood sample is allowed to clot at ambient temperature for approximately 30 minutes and then left for an additional 30 minutes at approximately 4 °C. Clotted samples are centrifuged for 10 minutes and the serum is aspirated and stored at -20 °C for analysis.

In the second week of the study, (approximately 1 week following the initial needleless injection), the animals are administered a Genotropin® formulation prepared as follows: 36 IU (approximately 30 mg) of the lyophilized Genotropin® powder is reconstituted into 1.8 mL of a suitable buffer without preservatives (e.g., sterile phosphate buffered saline (PBS)) to provide a Genotropin® solution having a concentration of 20 IU/mL. 1 mL dosages are withdrawn by syringe and gently mixed with 1 mL of buffer to provide an injection solution having a concentration of 10 IU/mL. Each animal is given an IV injection of 0.1 mL/kg of the solution in the left ear.

Following IV injection, multiple venous blood samples are taken from the marginal ear vein of the right ear at times 0, 5, 10, 30 minutes, 1, 2, 4, 6 and 12 hours after injection.

In the third week of the study, the animals are administered 0.1 mL/kg of a buffered Genotropin® solution (prepared as above) by sub-cutaneous injection. The injection site is observed to ensure that there is no leakage after administration. Following the SC injections, multiple venous blood samples are taken from the marginal ear vein of the right ear at times 0, 30 minutes, 1, 2, 4, 6, 12, 24 and 48 hours after administration.

In the fourth week of the study, approximately 0.8 mg powder/kg of densified Genotropin® particles (prepared as described in Example 1) are administered to each animal using a needleless syringe, and multiple venous blood samples are taken from the marginal ear vein of the right ear at times 0, 30 minutes, 1, 2, 4, 6, 12, 24 and 48 hours after administration.

Serum rhGH levels are determined as previously described and pharmacokinetic variables are calculated for each drug administration technique. It is expected that needleless syringe administration of the densified Genotropin® particles will result in achieving and maintaining *in vivo* therapeutic levels of the administered drug.

### Example 5

### Bio-Activity of Densified rhGH Delivered In Vivo to Growth Hormone-Deficient Rats

To evaluate the bio-activity of recombinant human growth hormone that has been densified using the method of the present invention the following study is carried out.

Dwarf or hypophysectomized (growth hormone-deficient) rats are administered pharmaceutical preparations containing either: densified Genotropin® particles; lyophilized Genotropin® powder; or placebo at approximately 4 IU rhGH per animal/week by daily sub-cutaneous (SC) injection. In particular, on 5 successive days, fur from the peritoneal region of the animal subjects is clipped prior to administration of the densified rhGH, the lyophilized rhGH or the placebo by SC injection. Body weight and, if desired, bone size and length are monitored on a daily basis.

Bio-activity of the densified rhGH particle formulation is determined by measuring body weight change over time. It is expected that the densified rhGH particle formulation will retain sufficient bio-activity

### Example 6

### Densification of Commonly Used Excipients

Finely ground powders of pharmaceutical grade mannitol and lactose were obtained and reprocessed using the method of the invention. Particularly, approximately 30 to 50 mg of mannitol or lactose were compacted under pressure using a Carver Laboratory Pellet Press (Model 3620, available from Carver, Inc., Wabash, IN). The pressure of compaction was 7.03 x 10⁵ g/cm² (10,000 lbs/in²) which was applied for approximately 30 seconds. The resulting compacted pellets were ground using mortar and pestle until visually broken up, and then sieved to select for particles having a size greater than about 50 *µ*m using the methods described in Example 1. In both the mannitol and the lactose preparations, a significant size reduction was observed when the compacted particles were compared against_like weights of the non-densified starting materials.

### Example 7

### Quantification of Densified Excipients

Pharmaceutical grade trehalose and mannitol excipients were obtained and processed according to the method of the invention. Both excipient preparations were processed in several different ways, and absolute density, envelope density, and average particle size of the resultant preparations were measured as described below.

Trehalose 45H3830 (Sigma) and mannitol K91698380-703 (Merck) were either sieved, or freeze dried, compacted, ground and then sieved. A range of the resulting preparations were then analyzed for particle size and density measurements.

Portions of each sugar excipient were sieved to obtain preparations having reduced particle sizes. Sieving was carried out using stainless steel sieves for two hours at 3 mm amplitude using three sieve sizes (75*µ*m, 53 *µ*m and 38 *µ*m) without additional processing.

Alternatively, portions of each sugar excipient were processed using the methods of the invention as follows. 40g of each of the sugars was dissolved in water, flash frozen, and then freeze dried over night. Samples of each freeze dried preparation were retained, and the remainder compacted in a 13 mm compression die (1.05x10⁶ g/cm² (15,000 lbs/inch²) for 45 seconds) into discs. The mannitol discs were ground using mortar and pestle, and then sieved as described above at 3 mm amplitude, using three sieve sizes. The trehalose discs were first ground in a vibratory ball mill (Retsch mill), then ground by mortar and pestle and sieved as above.

Samples from each of the above-described excipient preparations were then analyzed for absolute and envelope density. Absolute density was determined using helium pycnometry, and envelope density was determined using a GeoPyc™ Model 1360 Envelope Density Analyzer (Micromeritics Instrument Corp.). The results of the analysis are depicted below in Table 1.

**Table 1**

| Pretreatment | Trehalose Density (g/cm³) | | Mannitol Density (g/cm³) | |
|---|---|---|---|---|
| | Absolute | Envelope | Absolute | Envelope |
| Sieved | 1.5 | 0.5 | 1.5 | 0.5 |
| Freeze Dried | 1.5 | 0.3 | 1.5 | 0.3 |
| Freeze Dried, Compressed, Milled, Sieved | 1.5 | 0.8 | 1.5 | 0.8 |

As can be seen in Table 1, none of the various processing methods had a significant effect on the absolute density of the powdered excipients. Further, as expected, the non-compacted, freeze-dried sugars had a much lower envelope density than the other preparations, and a concomitantly higher porosity (measurement not shown). The density measurements for the trehalose and mannitol samples clearly demonstrate that the methods of the invention (compression, milling, sieving) lead to a significant increase in envelope density relative to both the freeze-dried and the sieved preparations. These results indicate that the novel methods described herein can be used to provide densified particulate pharmaceutical preparations that are suitable for delivery via needleless injection techniques.

Accordingly, novel methods for densifying particulate pharmaceutical compositions, and densified pharmaceutical compositions formed therefrom, have been described. Although preferred embodiments of the subject invention have been described in some detail, it is understood that obvious variations can be made without departing from the scope of the invention as defined by the appended claims.

## Claims

1. A method for forming densified particles from a particulate pharmaceutical preparation, comprising compacting the preparation to provide a compacted pharmaceutical preparation and size-reducing the compacted preparation into densified particles of suitable size and density for transdermal delivery thereof by needleless injection.

2. A method according to claim 1, wherein the densified particles have a size in the range of 0.1 to 150 µm.

3. A method according to claim 2, wherein the range is 20 to 60 µm.

4. A method according to any one of the preceding claims, wherein the densified particles have a particle density in the range of 0.8 to 3.0 g/cm³.

5. A method according to claim 4, wherein the range is 0.8 to 1.5 g/cm³.

6. A method according to any one of the preceding claims, wherein the particulate pharmaceutical preparation is a lyophilized or spray-dried composition.

7. A method according to any one of the preceding claims, wherein the compaction is carried out in a press at 7.03 x 10⁴ to 1.68 x 10⁶ g/cm² (1,000 to 24,000 pounds per square inch).

8. A method according to claim 7, wherein the compaction is carried out under vacuum.

9. A method according to any one of the preceding claims, wherein the compacting is carried out without heating or shear.

10. A method according to any one of the preceding claims, wherein the size reduction of the compacted material is carried out by milling and/or sieving.

11. A method according to any one of the preceding claims wherein the particulate pharmaceutical preparation is a preparation of a peptide or protein.

12. A method according to claim 11, wherein the peptide or protein is selected from insulin, calcitonin, octreotide, endorphin, liprecin, pituitary hormones, follicle luteoids, growth factors, asparaginase, chorionic gonadotropin, corticotropin and analogues thereof, erythropoitein, epoprostenol, glucagon, interferons, interleukins, menotropins, oxytocin, streptokinase, tissue plasminogen activator, urokinase, vasopressin, desmopressin, angiotensin II antagonists, antidiuretic hormone agonists, bradykinin antagonists, CD4 molecules, antibody molecules and antibody fragments, neurotrophic factors, colony stimulating factors, parathyroid hormone and agonists, parathyroid hormone antagonists, prostaglandin antagonists, protein C, protein S, renin inhibitors, thrombolytics, tumor necrosis factor, peptide vaccines, vasopressin antagonists and α-1 antitrypsin.

13. A method according to claim 12, wherein the peptide or protein is selected from human growth hormone, HMG, desmopressin acetate, growth factor releasing factor, somatostatin, somatotrophin, platelet-derived growth factor, urofollitropin follicle-stimulating hormone, luteinizing hormone, insulin-like growth factor-1 and Fab, Fab₂, Fv and sFv antibody fragments.

14. A method according to any one of claims 1 to 10, wherein the particulate pharmaceutical preparation is a preparation of a gene construct.

15. A densified particulate pharmaceutical composition formed from a lyophilized or spray-dried pharmaceutical preparation, said densified composition having an average particle size in the range of 0.1 to 250 µm and a particle density in the range of 0.1 to 25 g/cm³.

16. A composition according to claim 15, wherein the lyophilized or spray-dried pharmaceutical preparation is a heat-sensitive biopharmaceutical preparation.

17. A composition according to claim 15 or 16, wherein the lyophilised or spray-dried pharmaceutical preparation is a preparation of a peptide or protein.

18. A composition according to claim 17, wherein the peptide or protein is selected from insulin, calcitonin, octreotide, endorphin, liprecin, pituitary hormones, follicle luteoids, growth factors, asparaginase, chorionic gonadotropin, corticotropin and analogues thereof, erythropoitein, epoprostenol, glucagon, interferons, interleukins, menotropins, oxytocin, streptokinase, tissue plasminogen activator, urokinase, vasopressin, desmopressin, angiotensin II antagonists, antidiuretic hormone agonists, bradykinin antagonists, CD4 molecules, antibody molecules and antibody fragments, neurotrophic factors, colony stimulating factors, parathyroid hormone and agonists, parathyroid hormone antagonists, prostaglandin antagonists, protein C, protein S, renin inhibitors, thrombolytics, tumor necrosis factor, peptide vaccines, vasopressin antagonists and α-1 antitrypsin.

19. A composition according to claim 18, wherein the peptide or protein is selected from human growth hormone, HMG, desmopressin acetate, growth factor releasing factor, somatostatin, somatotrophin, platelet-derived growth factor, urofollitropin follicle-stimulating hormone, luteinizing hormone, insulin-like growth factor-1 and Fab, Fab₂, Fv and sFv antibody fragments.

20. A composition according claim 15 or 16, wherein the lyophilised or spray-dried pharmaceutical preparation is a preparation of a gene construct.

21. A composition according to any one of claims 15 to 20, wherein the particle size range is 0.1 to 150 µm.

22. A composition according to claim 21, wherein the range is 20 to 60 µm.

23. A composition according to any one of claims 15 to 22, wherein the particle density range is 0.8 to 3.0 g/cm³.

24. A composition according to claim 23, wherein the range is 0.8 to 1.5 g/cm³.

25. A needleless syringe loaded with particles produced by a method as defined in any one of claims 1 to 14 or with a composition as defined in any one of claims 15 to 24.

## Patentansprüche

1. Verfahren zur Herstellung verdichteter Partikel aus einer partikulären pharmazeutischen Präparation, umfassend das Kompaktieren der Präparation zum Erhalt einer kompaktierten pharmazeutischen Präparation und das Größenreduzieren der kompaktierten Präparation zu verdichteten Partikeln einer geeigneten Größe und Dichte zur transdermalen Verabreichung mittels nadelloser Injektion.

2. Verfahren nach Anspruch 1, wobei die verdichteten Partikel eine Größe im Bereich von 0,1 bis 150 µm aufweisen.

3. Verfahren nach Anspruch 2, wobei der Bereich 20 bis 60 µm beträgt.

4. Verfahren nach einem der vorangehenden Ansprüche, wobei die verdichteten Partikel eine Partikeldichte im Bereich von 0,8 bis 3,0 g/cm³ aufweisen.

5. Verfahren nach Anspruch 4, wobei der Bereich 0,8 bis 1,5 g/cm³ beträgt.

6. Verfahren nach einem der vorangehenden Ansprüche, wobei die partikuläre pharmazeutische Präparation eine lyophilisierte oder sprühgetrocknete Zusammensetzung ist.

7. Verfahren nach einem der vorangehenden Ansprüche, wobei die Kompaktierung in einer Presse bei 7,03 x 10⁴ bis 1,68 x 10⁶ g/cm² (1.000 bis 24.000 pounds pro inch²) vorgenommen wird.

8. Verfahren nach Anspruch 7, wobei die Kompaktierung unter Vakuum vorgenommen wird.

9. Verfahren nach einem der vorangehenden Ansprüche, wobei die Kompaktierung ohne Wärme oder Scherung vorgenommen wird.

10. Verfahren nach einem der vorangehenden Ansprüche, wobei die Größenreduzierung des kompaktierten Materials durch Mahlen und/oder Sieben vorgenommen wird.

11. Verfahren nach einem der vorangehenden Ansprüche, wobei die partikuläre pharmazeutische Präparation eine Präparation eines Peptids oder Proteins ist.

12. Verfahren nach Anspruch 11, wobei das Peptid oder Protein gewählt ist aus Insulin, Calcitonin, Octreotid, Endorphin, Liprecin, Hypophysenhormonen, Follikel-Luteoiden, Wachstumsfaktoren, Asparaginase, Choriongonadotropin, Corticotropin und deren Analoga, Erythropoetin, Epoprostenol, Glucagon, Interferonen, Interleukinen, Menotropinen, Oxytocin, Streptokinase, Gewebe-Plasminogenaktivator, Urokinase, Vasopressin, Desmopressin, Angiotensin-II-Antagonisten, antidiueritsche Hormon-Agonisten, Bradykinin-Antagonisten, CD4-Moleküle, Antikörper-Moleküle und Antikörper-Fragmente, neurotrophische Faktoren, Kolonie-stimulierende Faktoren, Nebennierenhormone und Agonisten, Nebennierenhormon-Antagonisten, Prostaglandin-Antagonisten, Protein C, Protein S, Renin-Inhibitoren, Thrombolytika, Tumor-Nekrose-Faktor, Peptid-Vakzine, Vasopressin-Antagonisten und α-1-Antitrypsin.

13. Verfahren nach Anspruch 12, wobei das Peptid oder Protein gewählt ist aus humanem Wachstumshormon, HMG, Desmopressinacetat, Wachstumshormon-Freisetzungsfaktor, Somatostatin, Somatotrophin, Thrombozyten-Wachstumsfaktor, Urofollitropin-Follikel-stimulierendes Hormon, luteinisierendes Hormon, Insulin-artiger Wachstumsfaktor 1 und Fab-, Fab₂-, Fv- und sFv-Antikörper-Fragmente.

14. Verfahren nach einem der Ansprüche 1 bis 10, wobei die partikuläre pharmazeutische Präparation eine Präparation aus einem Gen-Konstrukt ist.

15. Verdichtete partikuläre pharmazeutische Zusammensetzung, die aus einer lyophilisierten oder sprühgetrockneten Präparation hergestellt wird, wobei die verdichtete Zusammensetzung eine mittlere Teilchengröße im Bereich von 0,1 bis 250 µm und eine Partikeldichte im Bereich von 0,1 bis 25 g/cm³ aufweist.

16. Zusammensetzung nach Anspruch 15, wobei die lyophilisierte oder sprühgetrocknete pharmazeutische Präparation eine wärmeempfindliche biopharmazeutische Präparation ist.

17. Zusammensetzung nach Anspruch 15 oder 16, wobei die lyophilisierte oder sprühgetrocknete pharmazeutische Präparation eine Präparation aus einem Peptid oder Protein ist.

18. Zusammensetzung nach Anspruch 17, wobei das Peptid oder Protein gewählt ist aus Insulin, Calcitonin, Octreotid, Endorphin, Liprecin, Hypophysenhormonen, Follikel-Luteoiden, Wachstumsfaktoren, Asparaginase, Choriongonadotropin, Corticotropin und deren Analoga, Erythropoetin, Epoprostenol, Glucagon, Interferonen, Interleukinen, Menotropinen, Oxytocin, Streptokinase, Gewebe-Plasminogenaktivator, Urokinase, Vasopressin, Desmopressin, Angiotensin-II-Antagonisten, antidiueritsche Hormon-Agonisten, Bradykinin-Antagonisten, CD4-Moleküle, Antikörper-Moleküle und Antikörper-Fragmente, neurotrophische Faktoren, Kolonie-stimulierende Faktoren, Nebennierenhormone und Agonisten, Nebennierenhormon-Antagonisten, Prostaglandin-Antagonisten, Protein C, Protein S, Renin-Inhibitoren, Thrombolytika, Tumor-Nekrose-Faktor, Peptid-Vakzine, Vasopressin-Antagonisten und α-1-Antitrypsin.

19. Zusammensetzung nach Anspruch 18, wobei das Peptid oder Protein gewählt ist aus humanem Wachstumshormon, HMG, Desmopressinacetat, Wachstumshormon-Freisetzungsfaktor, Somatostatin, Somatotrophin, Thrombozyten-Wachstumsfaktor, Urofollitropin-Follikel-stimulierendes Hormon, luteinisierendes Hormon, Insulin-artiger Wachstumsfaktor 1 und Fab-, Fab₂-, Fv-und sFv-Antikörper-Fragmente.

20. Zusammensetzung nach Anspruch 15 oder 16, wobei die lyophilisierte oder sprühgetrocknete pharmazeutische Präparation eine Präparation aus einem Gen-Konstrukt ist.

21. Zusammensetzung nach einem der Ansprüche 15 bis 20, wobei die Partikelgröße im Bereich von 0,1 bis 150 µm liegt.

22. Zusammensetzung nach Anspruch 21, wobei der Bereich 20 bis 60 µm beträgt.

23. Zusammensetzung nach einem der Ansprüche 15 bis 22, wobei die Partikeldichte im Bereich von 0,8 bis 3,0 g/cm³ liegt.

24. Zusammensetzung nach Anspruch 23, wobei der Bereich 0,8 bis 1,5 g/cm³ beträgt.

25. Nadellose Spritze, befüllt mit Partikeln, die mittels einer Methode nach einem der Ansprüche 1 bis 14 oder mit einer Zusammensetzung nach einem der Ansprüche 15 bis 24 erzeugt sind.

## Revendications

1. Méthode pour former des particules densifiées à partir d'une préparation pharmaceutique particulaire, comprenant les étapes consistant à compacter la préparation pour obtenir une préparation pharmaceutique compactée et à réduire la taille de la préparation compactée en particules densifiées de taille et de densité appropriées à l'administration transdermique de celle-ci par une injection sans aiguille.

2. Méthode selon la revendication 1, dans laquelle les particules densifiées ont une taille dans l'intervalle allant de 0,1 à 150 *µ*m.

3. Méthode selon la revendication 2, dans laquelle l'intervalle va de 20 à 60 *µ*m.

4. Méthode selon l'une quelconque des revendications précédentes, dans laquelle les particules densifiées ont une densité de particule dans l'intervalle allant de 0,8 à 3,0 g/cm³.

5. Méthode selon la revendication 4, dans laquelle l'intervalle va de 0,8 à 1,5 g/cm³.

6. Méthode selon l'une quelconque des revendications précédentes, dans laquelle la préparation pharmaceutique particulaire est une composition lyophilisée ou séchée par atomisation.

7. Méthode selon l'une quelconque des revendications précédentes, dans laquelle le compactage est effectué dans une presse à 7,03 x 10⁴ à 1,68 x 10⁶ g/cm² (1000 à 24000 livres par pouce carré).

8. Méthode selon la revendication 7, dans laquelle le compactage est effectué sous vide.

9. Méthode selon l'une quelconque des revendications précédentes, dans laquelle le compactage est effectué sans chauffage ni cisaillement.

10. Méthode selon l'une quelconque des revendications précédentes, dans laquelle la réduction de taille du matériau compacté est effectuée par broyage et/ou tamisage.

11. Méthode selon l'une quelconque des revendications précédentes, dans laquelle la préparation pharmaceutique particulaire est une préparation d'un peptide ou d'une protéine.

12. Méthode selon la revendication 11, dans laquelle le peptide ou la protéine est choisi parmi l'insuline, la calcitonine, l'octréotide, l'endorphine, la liprécine, des hormones pituitaires, des lutéoïdes folliculaires, des facteurs de croissance, l'asparaginase, la gonadostimuline chorionique, la corticostimuline et des analogues de celle-ci, l'érythropoïétine, l'époprosténol, le glucagon, des interférons, des interleukines, des ménotropines, l'oxytocine, la streptokinase, l'activateur du plasminogène tissulaire, l'urokinase, la vasopressine, la desmopressine, des antagonistes d'angiotensine II, des agonistes d'hormone antidiurétique, des antagonistes de bradykinine, des molécules CD4, des molécules d'anticorps et des fragments d'anticorps, des facteurs neurotrophiques, des facteurs stimulant les colonies, l'hormone parathyroïdienne et des agonistes, des antagonistes d'hormone parathyroïdienne, des antagonistes de prostaglandine, la protéine C, la protéine S, des inhibiteurs de rénine, des agents thrombolytiques, le facteur nécrosant des tumeurs, des vaccins peptidiques, des antagonistes de vasopressine et l'antitrypsine α-1.

13. Méthode selon la revendication 12, dans laquelle le peptide ou la protéine est choisi parmi l'hormone de croissance humaine, les HMG, l'acétate de desmopressine, la somatocrinine, la somatostatine, la somatotrophine, le facteur de croissance dérivé des plaquettes, la folliculostimuline urofollitropine, l'hormone lutéinisante, le facteur de croissance 1 analogue à l'insuline, et les fragments d'anticorps Fab, Fab₂, Fv et sFv.

14. Méthode selon l'une quelconque des revendications 1 à 10, dans laquelle la préparation pharmaceutique particulaire est une préparation d'une construction de gène.

15. Composition pharmaceutique particulaire densifiée formée à partir d'une préparation pharmaceutique lyophilisée ou séchée par atomisation, ladite composition densifiée ayant une taille moyenne de particule comprise dans l'intervalle allant de 0,1 à 250 *µ*m et une densité de particule dans l'intervalle allant de 0,1 à 25 g/cm³.

16. Composition selon la revendication 15, dans laquelle la préparation pharmaceutique lyophilisée ou séchée par atomisation est une préparation biopharmaceutique thermosensible.

17. Composition selon la revendication 15 ou 16, dans laquelle la préparation pharmaceutique lyophilisée ou séchée par atomisation est une préparation d'un peptide ou d'une protéine.

18. Composition selon la revendication 17, dans laquelle le peptide ou la protéine est choisi parmi l'insuline, la calcitonine, l'octréotide, l'endorphine, la liprécine, des hormones pituitaires, des lutéoïdes folliculaires, des facteurs de croissance, l'asparaginase, la gonadostimuline chorionique, la corticostimuline et des analogues de celle-ci, l'érythropoïétine, l'époprosténol, le glucagon, des interférons, des interleukines, des ménotropines, l'oxytocine, la streptokinase, l'activateur du plasminogène tissulaire, l'urokinase, la vasopressine, la desmopressine, des antagonistes d'angiotensine II, des agonistes d'hormone antidiurétique, des antagonistes de bradykinine, des molécules CD4, des molécules d'anticorps et des fragments d'anticorps, des facteurs neurotrophiques, des facteurs stimulant les colonies, l'hormone parathyroïdienne et des agonistes, des antagonistes d'hormone parathyroïdienne, des antagonistes de prostaglandine, la protéine C, la protéine S, des inhibiteurs de rénine, des agents thrombolytiques, le facteur nécrosant des tumeurs, des vaccins peptidiques, des antagonistes de vasopressine et l'antitrypsine α-1.

19. Composition selon la revendication 18, dans laquelle le peptide ou la protéine est choisi parmi l'hormone de croissance humaine, les HMG, l'acétate de desmopressine, la somatocrinine, la somatostatine, la somatotrophine, le facteur de croissance dérivé des plaquettes, la folliculostimuline urofollitropine, l'hormone lutéinisante, le facteur de croissance 1 analogue à l'insuline, et les fragments d'anticorps Fab, Fab₂, Fv et sFv.

20. Composition selon la revendication 15 ou 16, dans laquelle la préparation pharmaceutique lyophilisée ou séchée par atomisation est une préparation d'une construction de gène.

21. Composition selon l'une quelconque des revendications 15 à 20, dans laquelle l'intervalle de taille de particule va de 0,1 à 150 *µ*m.

22. Composition selon la revendication 21, dans laquelle l'intervalle va de 20 à 60 *µ*m.

23. Composition selon l'une quelconque des revendications 15 à 22, dans laquelle l'intervalle de densité de particule va de 0,8 à 3,0 g/cm³.

24. Composition selon la revendication 23, dans laquelle l'intervalle va de 0,8 à 1,5 g/cm³.

25. Seringue sans aiguille chargée avec des particules produites par une méthode telle que définie dans l'une quelconque des revendications 1 à 14 ou avec une composition telle que définie dans l'une quelconque des revendications 15 à 24.
